# EUROPEAN PATENT SPECIFICATION

(11) **EP 0 560 622 B1**
(45) Date of publication and mention of the grant of the patent: **23.08.2000**
(21) Application number: 93301887.1
(22) Date of filing: 12.03.1993
(51) Int. Cl.: C07D 233/72, C07D 233/74

(54) **A process for producing 1-propargylimidazolidine-2,4-dione**
Verfahren zur Herstellung von 1-Propargylimidazolidin-2,4-Dion
Procédé pour la préparation de 1-propargylimidazolidin-2,4-dione

(30) Priority: 12.03.1992 JP 5355192
(43) Date of publication of application: 15.09.1993
(73) Proprietor: SUMITOMO CHEMICAL COMPANY LIMITED, Osaka-shi, Osaka 541-0041 (JP)
(72) Inventor: Ohtaka, Ken, Takarazuka-shi, Hyogo-ken (JP); Furukawa, Yoshio, Oita-shi, Oita-ken (JP)
(74) Representative: Cresswell, Thomas Anthony

(56) References cited:
- EP-A- 0 285 270
- US-A- 4 176 189
- JOURNAL OF MEDICINAL CHEMISTRY vol. 35, no. 17 , 21 August 1992 , WASHINGTON pages 3270 - 3279 BJÖRN M. NILSSON ET. AL.

## Description

The present invention relates to a process for producing 1-propargylimidazolidine-2,4-dione which is useful as an intermediate for the production of insecticides.

It is known that 1-propargylimidazolidine-2,4-dione represented by the formula 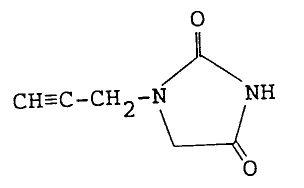 is an intermediate in the production of insecticidally active compounds. Its use in the production of insecticidal and acaricidal hydantoin N-methylol esters is described in US-A-4,176,189. The development of a commercially advantageous process for its production is therefore desired.

EP-A-0 285 270 describes a process for producing 1-propargylimidazolidine-2,4-dione which comprises reacting an alkyl ester of N-propargyl-N-alkoxycarbonylglycine with aqueous or gaseous ammonia to obtain a corresponding amide, and then further reacting the said amide with a base.

The present invention now provides a process for producing 1-propargylimidazolidine-2,4-dione, which process comprises
(i) reacting a compound of the formula (I) 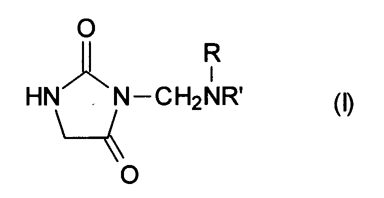 wherein R and R' are the same or different and each is (i) C₁-C₁₀ alkyl, (ii) phenyl or naphthyl, said phenyl or naphthyl being unsubstituted or substituted by 1 to 3 substituents selected from C₁-C₄ alkyl, halogen and cyano, or (iii) phenyl-substituted C₁-C₄ alkyl, or R and R' together form a C₃-C₈ alkylene group or a C₃-C₈ alkylene group which contains in its structure a hetero atom, optionally substituted by C₁-C₄ alkyl, with a compound of the formula (II)

   CH≡C-CH₂-X (II)

   wherein X is a halogen atom or a sulphonic acid residue of formula -OSO₂R^{a} wherein R^{a} is C₁-C₄ alkyl, tolyl or phenyl in the presence of a base selected from an alkali metal hydride, an alkali metal hydroxide and an alkali metal carbonate using a mixture of dimethyl formamide or dimethyl sulphoxide and an ether solvent or halogenated alkane solvent at a temperature of from 0°C to 100°C; and
(ii) treating the reaction mixture with an aqueous solution of hydrochloric acid or sulphuric acid at a temperature of from 0°C to 100°C.

In the definition of R and R' a C₁₋₁₀ alkyl group may be, for instance, methyl, ethyl, butyl or octyl. In the phenyl or naphthyl group the optional substituents include C₁-C₄ alkyl groups such as methyl and ethyl, halogen atoms such as fluorine, chlorine, bromine and iodine, and cyano groups. A phenyl-substituted C₁-C₄ alkyl group may be, for instance, benzyl, β-phenethyl or α-phenethyl. A C₃-C₈ alkylene group may be, for instance, trimethylene, tetramethylene or pentamethylene. A C₃₋₈ alkylene group containing in its structure a hetero atom may contain oxygen (-O-), sulfur (-S-) or nitrogen substituted by a C₁₋₄ alkyl group such as methyl or ethyl (-N(alkyl)-). Examples of a C₃₋₈ alkylene group containing such a hetero atom are CH₂CH₂OCH₂CH₂, CH₂CH₂SCH₂CH₂ and CH₂CH₂N(CH₃)CH₂CH₂.

In formula (II), the leaving group X is a halogen atom such as chlorine, bromine or iodine, or a sulfonic acid residue as defined above. Examples of a sulphonic acid residue include a methanesulfonic acid residue, p-toluenesulfonic acid residue and benzenesulfonic acid residue.

The base which is used in step (i) is an alkali metal hydride, such as sodium hydride or potassium hydride; an alkali metal hydroxide such as potassium hydroxide or sodium hydroxide; or an alkali metal carbonate such as sodium carbonate or potassium carbonate.

The reaction between the compound of the formula (I) and the compound of the formula (II) is conducted in a solvent, which comprises a mixture of dimethylformamide or dimethyl sulfoxide and an ether solvent such as tetrahydrofuran or dioxane, or a halogenated alkane such as methylene chloride or 1,2-dichloroethane. The inclusion of dimethylformamide or dimethyl sulphoxide, for instance in an amount of about 0.01 to about 10% (1:0.0001 to 1:0.1) based on the above-mentioned solvent by weight, leads to an improved reaction yield.

The reaction of step (i) is carried out at a temperature in the range of 0°C to 100°C, typically for about 1 to about 48 hours. The compound of formula (II) is used generally in an amount of about 1 to about 3 moles and the base is used in an amount of about 1 to about 3 moles, each per mole of the compound of the formula (I).

When the base used in this reaction is an alkali metal hydroxide such as potassium hydroxide or sodium hydroxide, or an alkali metal carbonate such as sodium carbonate or potassium carbonate, the reaction is preferably carried out in the presence of an auxiliary agent such as a phase transfer catalyst. Such an auxiliary agent is typically used in an amount of about 0.001 to about 0.2 mole per mole of the compound of the formula (I). Examples of the agent include, for instance, tertiary amines such as tris[2-(2-methoxyethoxy)ethyl]amine, tris-(3,6-dioxaheptyl)amine and tris(3,6-dioxaoctyl)amine, quaternary ammonium salts such as tetra-n-butylammonium bromide, benzylytriethylammonium chloride and tetra-n-butylammonium sulfate, crown ethers such as 18-crown-6 and dicyclohexano-18-crown-6, and polyethylene glycols such as polyethylene glycol (PEG) 400 and PEG 1540. These agents can be used either singly or in combination.

In the step (ii) of the process of the invention, the reaction mixture obtained by the reaction of the compound of the formula (I) with the compound of the formula (II) in the presence of the base is treated with an aqueous solution of hydrochloric acid or sulphuric acid to give 1-propargylimidazolidine-2,4-dione. The water in the aqueous solution added to the reaction mixture is preferably present in an amount of about 10 to about 500 parts by weight per 100 parts by weight of the reaction mixture. It is preferable to remove most (e.g. 70 - 99 vol. %) of the solvent from the reaction mixture prior to this acid treatment. The acid treatment is generally carried out by treating the reaction mixture with the aqueous solution of hydrochloric acid or sulfuric acid at a temperature of about 0 to about 100°C, preferably at room temperature, for about 1 minute to about 24 hours. The acid is used in an amount effective to adjust the pH of the resulting mixture to about 2 to about 4.

After this acid treatment, 1-propargylimidazolidine-2,4-dione can be isolated by a conventional procedure, for example by extracting the mixture with an organic solvent and removing the solvent under reduced pressure. If necessary, the product compound can be further purified, for instance by chromatography.

The compound of formula (I) for use in the present invention can be prepared, for example by reacting readily available imidazolidine-2,4-dione, formalin and a compound of the formula

RR'NH (III)

wherein R and R' are as defined above.

This reaction is generally carried out in a solvent. Suitable solvents include, for instance, alcohols such as methanol, ethanol and isopropyl alcohol, ketones such as acetone and methyl isobutyl ketone, ethers such as tetrahydrofuran and dioxane, dimethylformamide and dimethyl sulfoxide.

This reaction is generally conducted at a temperature between about -10°C and about 50°C for about 1 to about 24 hours. Per mole of imidazolidine-2,4-dione, formalin is used in an amount of about 0.8 to about 2 moles and the compound of the formula (III) is used in an amount of about 0.8 to about 10 moles.

After completion of the reaction, the compound of the formula (I) can be isolated and purified by a known procedure, for example by removing the reaction solvent under reduced pressure and, if required, subjecting the residue to chromatography or to crystallization from an organic solvent.

The following examples further illustrate the present invention.

### Example 1

A 60% dispersion of sodium hydride in oil was taken in an amount equivalent to 1.21 g (0.050 mole) of sodium hydride and suspended in 100 g of dry tetrahydrofuran. Then, the mixture was cooled to a temperature not exceeding 10°C, and 5.98 g (0.030 mole) of 3-morpholinomethylimidazolidine-2,4-dione and 1.0 g of dry dimethylformamide were added thereto with stirring. Then, the mixture was allowed to return to room temperature and further stirred at room temperature for 2 hours. To this reaction mixture was added dropwise 4.23 g (0.032 mole) of propargyl methanesulfonate at room temperature with stirring and the mixture was further stirred at room temperature for 20 hours.

To the resulting reaction mixture was added 80 g of water and after more than 90 % of the volume of tetrahydrofuran was removed under reduced pressure, the residue was washed with two 100 g portions of hexane. The aqueous layer was adjusted to pH about 2 with 30% sulfuric acid, stirred at room temperature for half an hour, saturated with sodium chloride and extracted with six 100 g portions of methyl isobutyl ketone. The methyl isobutyl ketone layers were combined and the solvent was then distilled off under reduced pressure to give 3.75 g (0.027 mole) of 1-propargylimidazolidine-2,4-dione.
Yield 90.5%
¹H-NMR δ (DMSO-D₆): 2.60 (1H, t, J=2 Hz), 3.98 (2H, s), 4.19 (2H, d, J=2 Hz), 7.64 (1H, bs)

### Example 2

A 60% dispersion of sodium hydride in oil was taken in an amount equivalent to 1.21 g (0.050 mole) of sodium hydride and suspended in 150 g of dry tetrahydrofuran. Then, the mixture was cooled to a temperature not exceeding 10°C, and 5.98 g (0.030 mole) of 3-morpholinomethylimidazolidine-2,4-dione and 0.1 g of dry dimethylformamide were added thereto with stirring. Then, the mixture was allowed to return to room temperature and further stirred at room temperature for 3 hours. To this reaction mixture was added dropwise 4.44 g (0.033 mole) of propargyl methanesulfonate at room temperature with stirring over a period of 10 minutes and the mixture was further stirred at room temperature for 20 hours and at 60°C for 6 hours.

Thereafter, the reaction mixture was treated in the same manner as in Example 1 to give 3.88 g (0.028 mole) of 1-propargylimidazolidine-2,4-dione.
Yield 93.6%

### Example 3

In 150 g of dry 1,2-dichloroethane are suspended 4.56 g (0.033 mole) of potassium carbonate and 40 mg (0.124 mmole) of tris(3,6-dioxaheptyl)amine, and after heating to 30°C, 5.98 g (0.030 mole) of 3-morpholinomethylimidazolidine-2,4-dione and 0.1 g of dry dimethylformamide are added thereto with stirring. The mixture is further stirred at 30° to 35°C for 3 hours. Then, 4.44 g (0.033 mole) of propargyl methanesulfonate is added dropwise to the above mixture over a period of 10 minutes at room temperature with stirring and the mixture is further stirred at 30 to 35°C for 20 hours.

Thereafter, the reaction mixture is treated in the same manner as in Example 1 to give 1-propargylimidazolidine-2,4-dione.

The following is a production example for the compound of the formula (I).

### Reference Example 1

In 7 ml of methanol was dissolved 100 mg (1.00 mmole) of imidazolidine-2,4-dione, and after cooling to a temperature not exceeding 10°C, 87.1 mg (1.00 mmole) of morpholine was added. To this mixture was added 81.2 mg (1.00 mmole) of 37% aqueous solution of formaldehyde (formalin) at a temperature not higher than 10°C and the mixture was stirred at 20 to 25°C for 3 hours. The solvent was then distilled off under reduced pressure, followed by addition of 3 ml of toluene, and the solvent was removed under reduced pressure. The residue was subjected to neutral alumina column chromatography to recover 159 mg (0.798 mmole) of 3-morpholinomethylimidazolidine-2,4-dione.
Yield 80%
Melting point 132-133°C

In accordance with the process of the present invention, 1-propargylimidazolidine-2,4-dione, a useful intermediate for the production of insecticidally active compounds, can be easily provided. The insecticidally active compounds can be prepared from 1-propargylimidazolidine-2,4-dione by the method described in U. S. Patent No. 4,176,189.

## Claims

1. A process for producing 1-propargylimidazolidine-2,4-dione, which process comprises
(i) reacting a compound of the formula (I) 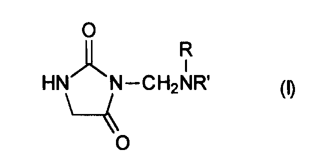 wherein R and R' are the same or different and each is (i) C₁-C₁₀ alkyl, (ii) phenyl or naphthyl, said phenyl or naphthyl being unsubstituted or substituted by 1 to 3 substitutents selected from C₁-C₄ alkyl, halogen and cyano, or (iii) phenyl-substituted C₁-C₄ alkyl, or R and R' together form a C₃-C₈ alkylene group or a C₃-C₈ alkylene group which contains in its chain structure a hetero atom, optionally substituted by C₁-C₄ alkyl, with a compound of the formula (II)
CH≡C-CH₂-X (II)
wherein X is a halogen atom or a sulphonic acid residue of formula -OSO₂R^{a} wherein R^{a} is C₁-C₄ alkyl, tolyl or phenyl, in the presence of a base selected from an alkali metal hydride, an alkali metal hydroxide and an alkali metal carbonate using a mixture of dimethylformamide or dimethyl sulphoxide and an ether solvent or a halogenated alkane solvent at a temperature of from 0°C to 100°C; and
(ii) treating the reaction mixture from step (i) with an aqueous solution of hydrochloric acid or sulphuric acid at a temperature of from 0°C to 100°C.

2. A process according to claim 1, wherein the mixing ratio of the ether solvent to dimethylformamide or dimethylsulfoxide is 1:0.0001 to 1:0.1 by weight.

3. A process according to claim 1 or 2 wherein the ether solvent is tetrahydrofuran.

4. A process according to any one of the preceding claims wherein the alkali metal hydride is sodium hydride.

5. A process according to any one of the preceding claims wherein the mixing ratio of the halogenated alkane solvent to dimethylformamide or dimethylsulfoxide is 1:0.0001 to 1:0.1 by weight.

6. A process solvent according to any one of the preceding claims wherein the halogenated alkane solvent is 1,2-dichloroethane.

7. A process according to any one of the preceding claims wherein the base is potassium carbonate.

8. A process according to any one of claims 1 to 3 and 5 to 7 wherein the base is an alkali metal hydroxide or carbonate and the reaction in step (i) is carried out in the presence of a phase transfer catalyst.

9. A process according to any one of claims 1 to 8 wherein the compound of formula (II) and the base are used in an amount of 1 to 3 moles and 1 to 3 moles, respectively, per mole of the compound of the formula (I).

10. A process according to any one of claims 1 to 9 wherein R and R' together represent -CH₂CH₂OCH₂CH₂-.

11. A process according to any one of claims 1 to 10 comprising producing the compound of formula (I) by reacting imidazolidine-2,4-dione with formalin and a compound of the formula (III)
RR'NH (III)
wherein R and R' are as defined in claim 1 in relation to formula (I).

## Patentansprüche

1. Verfahren zur Herstellung von 1-Propargylimidazolidin-2,4-dion, wobei das Verfahren umfaßt:
(i) Umsetzen einer Verbindung der Formel (I) 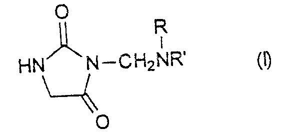 in der R und R' gleich oder verschieden sind und je einen (i) C₁-C₁₀ Alkylrest, (ii) eine Phenyl- oder Naphtylgruppe, wobei besagte Phenyl- oder Naphtylgruppe unsubstituiert oder mit 1 bis 3 Substituenten, ausgewählt aus C₁-C₄ Alkyl, Halogen und Cyano substituiert sein kann, oder (iii) einen phenylsubstituierten C₁-C₄ Alkylrest darstellen, oder R und R' zusammen einen C₃-C₈ Alkylenrest oder einen C₃-C₈ Alkylenrest, der in seiner Kettenstruktur ein Heteroatom enthält, gegebenenfalls durch einen C₁-C₄ Alkylrest substituiert, bilden, mit einer Verbindung der Formel (II)
CH≡O-CH₂-X (II)
wobei X ein Halogenatom oder ein Sulfonsäurerest der Formel -OSO₂R^{a} ist, in der R^{a} ein C₁-C₄ Alkyl-, ein Tolyl- oder Phenylrest ist, in Gegenwart einer Base, ausgewählt aus einem Alkalimetallhydrid, einem Alkalimetallhydroxid und einem Alkalimetallcarbonat, unter Verwendung eines Gemisches von Dimethylformamid oder Dimethylsulfoxid und eines Ether-Lösungsmittels oder eines halogenierten Alkan-Lösungsmittels bei einer Temperatur von 0°C bis 100°C; und
(ii) Behandlung des Reaktionsgemisches aus Schritt (i) mit einer wäßrigen Lösung von Salzsäure oder Schwefelsäure bei einer Temperatur von 0°C bis 100°C.

2. Verfahren nach Anspruch 1, wobei das Mischungsverhältnis des Ether-Lösungsmittels zu Dimethylformamid oder Dimethylsulfoxid bezogen auf das Gewicht 1:0,0001 bis 1:0,1 beträgt.

3. Verfahren nach Anspruch 1 oder 2, wobei das Ether-Lösungsmittel Tetrahydrofuran ist.

4. Verfahren nach einem der vorhergehenden Ansprüche, wobei das Alkalimetallhydrid Natriumhydrid ist.

5. Verfahren nach einem der vorhergehenden Ansprüche, wobei das Mischungsverhältnis des halogenierten Alkan-Lösungsmittels zu Dimethylformamid oder Dimethylsulfoxid bezogen auf das Gewicht 1:0,0001 bis 1:0,1 beträgt.

6. Verfahren nach einem der vorhergehenden Ansprüche, wobei das halogenierte Alkan-Lösungsmittel 1,2-Dichlorethan ist.

7. Verfahren nach einem der vorhergehenden Ansprüche, wobei die Base Kaliumcarbonat ist.

8. Verfahren nach einem der Ansprüche 1 bis 3 und 5 bis 7, wobei die Base ein Alkalimetallhydroxid oder -carbonat ist und der Reaktionsschritt (i) in Gegenwart eines Phasentransferkatalysators durchgeführt wird.

9. Verfahren nach einem der Ansprüche 1 bis 8, wobei die Verbindung der Formel (II) und die Base in einer Menge von 1 bis 3 Mol bzw. 1 bis 3 Mol pro Mol der Verbindung der Formel (I) eingesetzt werden.

10. Verfahren nach einem der Ansprüche 1 bis 9, wobei R und R' zusammen -CH₂CH₂OCH₂CH₂- bedeuten.

11. Verfahren nach einem der Ansprüche 1 bis 10, umfassend die Herstellung der Verbindung der Formel (I) durch Umsetzen von Imidazolidin-2,4-dion mit Formalin und einer Verbindung der Formel (III),
RR'NH (III)
in der R und R' wie in Anspruch 1 in Bezug auf Formel (I) definiert sind.

## Revendications

1. Procédé pour la production de 1-propargylimidazolidine-2,4-dione, lequel procédé comprend :
**(i)** la réaction d'un composé de formule (I) 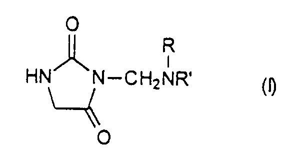 dans laquelle R et R' sont identiques ou différents et chacun d'eux représente (i) un groupe alkyle C₁-C₁₀, (ii) un groupe phényle ou naphtyle, ledit groupe phényle ou naphtyle étant non substitué ou substitué par 1 à 3 substituants choisis parmi les groupes alkyle C₁-C₄, halogènes et cyano, ou (iii) un groupe alkyle C₁-C₄ substitué par des groupes phényle, ou bien R et R' en association, forment un groupe alkylène C₃-C₈ ou un groupe alkylène contenant un hétéroatome dans sa structure, éventuellement substitué par un groupe alkyle C₁-C₄, avec un composé de formule (II)
CH≡C-CH₂-X (II)
dans laquelle X représente un halogène ou un résidu d'acide sulfonique de formule -OSO₂R^{a} dans laquelle R^{a} est un groupe alkyle C₁-C₄, tolyle ou phényle,
en présence d'une base choisie parmi un hydrure de métal alcalin, un hydroxyde de métal alcalin et un carbonate de métal alcali en utilisant un mélange de diméthylformamide ou diméthylsulfoxyde et d'un solvant ester ou d'un solvant alcane halogéné à une température comprise entre 0°C et 100°C ; et
**(ii)** le traitement du mélange réactionnel provenant de l'étape **(i)** avec une solution aqueuse d'acide chlorhydrique ou d'acide sulfurique à une température comprise entre 0°C et 100°C.

2. Procédé selon la revendication 1, dans lequel le rapport pondéral de mélange solvant éther/diméthylformamide ou diméthylsulfoxyde est compris entre 1/0,0001 et 1/0,1.

3. Procédé selon la revendication 1 ou la revendication 2, dans lequel le solvant éther est le tétrahydrofurane.

4. Procédé selon l'une quelconque des revendications précédentes, dans lequel l'hydrure de métal alcalin est l'hydrure de sodium.

5. Procédé selon l'une quelconque des revendications précédentes, dans lequel le rapport pondéral de mélange solvant alcane halogéné/diméthylformamide ou diméthylsulfoxyde est compris entre 1/0,0001 et 1/0,1.

6. Procédé selon l'une quelconque des revendications précédentes, dans lequel le solvant halogéné est le 1,2-dichloroéthane.

7. Procédé selon l'une quelconque des revendications précédentes, dans lequel la base est le carbonate de potassium.

8. Procédé selon l'une quelconque des revendications 1 à 3 et des revendications 5 à 7, dans lequel la base est un hydroxyde ou un carbonate de métal alcalin, et la réaction de l'étape **(i)** est effectuée en la présence d'un catalyseur de transfert de phase.

9. Procédé selon l'une quelconque des revendications 1 à 8, dans lequel le composé de formule (II) et la base sont utilisés respectivement en quantités comprises entre 1 et 3 moles et 1 et 3 moles par mole de composé de formule (I).

10. Procédé selon l'une quelconque des revendications 1 à 9, dans lequel R et R' associés représentent -CH₂CH₂OCH₂CH₂-.

11. Procédé selon l'une quelconque des revendications 1 à 10, comprenant la production du composé de formule (I) par la réaction de l'imidazolidine-2,4-dione avec le formol et un composé de formule (III)
RR'NH (III)
dans laquelle R et R' sont tels que définis dans la revendication 1 concernant la formule (I).
